# EUROPÄISCHE PATENTSCHRIFT

(11) **EP 1 073 628 B1**
(45) Veröffentlichungstag und Bekanntmachung des Hinweises auf die Patenterteilung: **01.10.2003**
(21) Anmeldenummer: 99920644.4
(22) Anmeldetag: 12.04.1999
(51) Int. Cl.: C07C 263/10, C07C 263/20, C07C 265/14

(54) **VERFAHREN ZUR HERSTELLUNG VON MISCHUNGEN AUS DIPHENYLMETHANDIISOCYANATEN UND POLYPHENYLEN-POLYMETHYLEN-POLYISOCYANATEN MIT VERMINDERTEM GEHALT AN CHLORIERTEN NEBENPRODUKTEN UND VERMINDERTER JODFARBZAHL**
METHOD FOR PRODUCING MIXTURES CONSISTING OF DIPHENYLMETHANE DIISOCYANATES AND POLYPHENYLENE-POLYMETHYLENE-POLYISOCYANATES CONTAINING A REDUCED AMOUNT OF CHLORINATED SECONDARY PRODUCTS AND WITH A REDUCED IODINE COLOUR INDEX
PROCEDE POUR LA PREPARATION DE MELANGES DE DIPHENYLMETHANE-DIISOCYANATES ET DE POLYISOCYANATES DE POLYPHENYLENE-POLYMETHYLENE A TENEUR REDUITE EN SOUS-PRODUITS CHLORES ET A INDICE COLORIMETRIQUE D'IODE REDUIT

(30) Priorität: 21.04.1998 DE 19817691
(43) Veröffentlichungstag der Anmeldung: 07.02.2001
(73) Patentinhaber: BASF AKTIENGESELLSCHAFT, 67056 Ludwigshafen (DE)
(72) Erfinder: PENZEL, Ulrich, D-01945 Tettau (DE); SCHARR, Volker, D-01968 Senftenberg (DE); STAROSTA, Dieter, D-01987 Schwarzheide (DE); BOESEL, Hilmar, D-01968 Senftenberg (DE); STRÖFER, Eckhard, D-68163 Mannheim (DE); PFEFFINGER, Joachim, D-67063 Ludwigshafen (DE); POPLOW, Frank, D-51491 Overath (DE); DOSCH, Jürgen, D-67063 Ludwigshafen (DE); SCHWARZ, Hans, Volkmar, B-1410 Waterloo (BE); NÄUMANN, Fritz, D-64625 Bensheim (DE); VAN DEN ABEEL, Peter, B-2950 Kapellen (BE); JACOBS, Jan, NL-4631 LN Hoogerheide (NL); NEVEJANS, Filip, B-9170 St. Pauwels (BE); VAN PEE, Willy, B-1180 Bruxelles (BE)
(86) Internationale Anmeldenummer: EP9902453
(87) Internationale Veröffentlichungsnummer: WO99054289

(56) Entgegenhaltungen:
- EP-A- 0 445 602
- EP-A- 0 751 118
- DE-C- 3 744 001
- US-A- 3 381 025

## Beschreibung

Erfindungsgegenstand ist ein Verfahren zur Herstellung von Mischungen aus Diphenylmehandiisocyanaten und Polyphenylen-polymethylen-polyisocyanaten, sogenanntes PMDI, mit vermindertem Gehalt an chlorierten Nebenprodukten und verminderter Jodfarbzahl durch zweistufige Umsetzung der entsprechenden Mischungen aus Diphenylmethan-diaminen und Polyphenylen-polymethylen-polyaminen, sogenanntes PMDA, mit Phosgen in Gegenwart mindestens eines inerten organischen Lösungsmittels, wobei die in der ersten Stufe der Phosgenierung gebildeten entsprechenden Carbamylchloride und Aminhydrochloride in der zweiten Stufe der Phosgenierung einen Verweilzeitapparat durchlaufen, in dem die Aminhydrochloride zu den entsprechenden Carbamylchloriden phosgeniert und die Carbamylchloride zu den entsprechenden Isocyanaten und Chlorwasserstoff gespalten werden und die Massenverhältnisse von Phosgen zu Chlorwasserstoff gleichzeitig in der Flüssigphase 10-30:1 und in der Gasphase 1-10:1 betragen.

PMDI ist das technisch bedeutendste Isocyanat zur Herstellung von Polyurethan-Hartschaumstoffen, die bevorzugt als Dämmaterial in der Bauindustrie, als Isolierschaumstoff in der Kühlmöbelindustrie und als Sandwich-Konstruktionswerkstoff eingesetzt werden. Üblicherweise wird aus dem PMDI ein Teil des darin enthaltenen 4,4'-Diphenylmethan-diisocyanats, sogenanntes MMDI, durch eine geeignete technologische Operation, wie z.B. Destillation oder Kristallisation, gewonnen. MMDI seinerseits ist wichtiger Bestandteil von Polyurethanformulierungen kompakter, mikrozellularer und zelliger Polyurethane, wie z.B. Klebstoffe, Beschichtungen, Fasern, Elastomere, Integralschaumstoffe. Durch den Begriff "PMDI" in dieser Schrift sind demnach auch PMDI-Mischungen definiert, die monomeres MDI, beispielsweise 4,4'-, 2,2'- und/oder 2,4'-MDI enthalten.

PMDI wird bekanntermaßen hergestellt durch Phosgenierung des entsprechenden PMDA in Gegenwart eines inerten organischen Lösungsmittels. PMDA seinerseits wird durch eine saure Anilin-Formaldehyd-Kondensation erhalten, wobei sie technisch sowohl kontinuierlich als auch diskontinuierlich ausgeführt sein kann. Durch die Wahl der Mengenverhältnisse von Anilin, Formaldehyd und saurem Katalysator sowie eines geeigneten Temperatur- und Verweilzeitprofils werden die Anteile an Diphenylmethandiaminen und den homologen Polyphenylen-polymethylen-polyaminen sowie deren Stellungsisomeren im PMDA gesteuert. Große Gehalte an 4,4'-Diphenylmethandiamin bei einem gleichzeitig geringen Anteil des 2,4'-Isomers des Diphenylmethandiamins werden im technischen Maßstab durch die Verwendung starker Mineralsäuren, wie z.B. Salzsäure, als Katalysator der Anilin-Formaldehyd-Kondensation erhalten.

Allen in der Fach- und Patentliteratur beschriebenen sauren Anilin-Formaldehyd-Kondensationsverfahren ist die Bildung unerwünschter Nebenprodukte, wie z.B. die Bildung N-methylierter und N-formylierter Verbindungen sowie die Bildung von Dihydrochinazolinen, gemeinsam. Weiterhin können technische PMDA Restmengen nicht umgelagerter Aminobenzylaniline enthalten, die ihrerseits wieder Ausgangspunkt weiterer Reaktionen sein können. Nachteilig ist weiterhin, daß bei der sauren Anilin-Formaldehyd-Kondensation Chromophore gebildet werden, die das PMDA verfärben. Diese Verfärbungen werden nach der sauren Kondensation bei der nachfolgenden Neutralisation des sauren Kondensationskatalysators und der Entfernung des bei der Kondensation im Überschuß eingesetzten Anilins sowie in den anschließenden Verfahrensstufen der PMDI-Herstellung nicht oder nur unzureichend vermindert.

In der Phosgenierungsstufe wird das PMDA mit Phosgen in einem inerten organische Lösungsmittel zu PMDI umgesetzt. Die unerwünschten Nebenprodukte und Chromophore im PMDA können mit Phosgen zu weiteren Verbindungen, wie z.B. sekundären Carbamylchloriden und Chlorierungsprodukten am aromatischen Kern und/oder an der Methylenbrücke, reagieren. Zusätzlich entstehen in der Phosgenierungsstufe weitere chlortragende Nebenverbindungen, wie z.B. Allophanylchloride und Isonitrildichloride. Die chlortragenden Verbindungen und Chromophore sind sowohl in der niedermolekularen Fraktion, deren wesentlicher Bestandteil das Diphenylmethan-diisocyanat ist, als auch in den oligomeren Fraktionen des Polyphenylen-polymethylen-polyisocyanats incorporiert.

Die der Phosgenierung folgenden technologischen Operationen der Entfernung des im Überschuß eingesetzten Phosgens, der Entfernung des inerten Lösungsmittels, der thermischen Behandlung, der sogenannten Entchlorierung, und der Entfernung eines Teils des im Roh-PMDI enthaltenen MMDI durch Destillation und/oder Kristallisation vermindern den Gehalt an chlortragenden Verbindungen nicht nachhaltig und die Verfärbung des Roh-PMDI nimmt mit fortschreitender, vor allem thermischer Belastung des Produktes zu.

Chlorhaltiges und/oder verfärbtes PMDI ist bei der Weiterverarbeitung zu Polyisocyanat-Polyalkohol-Polyadditionskunststoffen unerwünscht. Insbesondere können chlorhaltige Verbindungen, die im Sinne der Bestimmungsmethode nach ASTM D 1638-74 ionisches Chlorid leicht bilden können, die Treibreaktion der Schaumstoffherstellung durch Salzbildung mit dem Treibkatalysator erheblich stören. Unerwünschte Verfärbungen des PMDI sind auch in den aus ihnen hergestellten Kunststoffen wirksam. Obwohl die Eigenfarbe der Polyisocyanat-Polyalkohol-Polyadditionskunststoffe deren mechanische Eigenschaften nicht negativ beeinflußt, sind helle Produkte wegen deren guten Variabilität im Produktionsprozeß des Verarbeiters, z.B. hinsichtlich Durchscheinen durch dünne Deckschichten und farbliche Gestaltungsmöglichkeiten, bevorzugt.

Es hat daher nicht an Versuchen gefehlt, den Gehalt an chlorierten Nebenprodukten und die Verfärbungen des PMDI in Mischungen mit MMDI zu vermindern.

Nach Angaben der GB 1.549.294 kann durch Zugabe von Isoharnstoffen in einer Menge von 25 - 250 Mol-% zur Acidität nach ASTM D 1638-74 des PMDI dieser Parameter reduziert werden. Nachteilig dabei ist, daß ein zusätzliches Agens verwendet werden muß und die Senkung der Acidität nur unvollständig gelingt.

In DD 285.593 wird die Behandlung von PMDI mit Säureamiden in einer Menge von 0,01-0,2 % bei 100-140°C innerhalb von 0,2-6 Stunden vorgeschlagen. Nach der Behandlung wird der gebildete Chlorwasserstoff durch Strippen mit Stickstoff oder Lösungsmitteldämpfen ausgetrieben. Nachteilig sind bei diesem Verfahren die unzureichende Wirkung der Säureamide, die Bildung zusätzlicher Inhaltsstoffe im PMDI durch die nicht vermeidbare Nebenreaktion der Isocyanate mit den Säureamiden zu acylierten Harnstoffen sowie der apparative Aufwand für die Behandlung des PMDI mit den Säureamiden und dem Ausstrippen des als Katalysator zugesetzten sowie des gebildeten Chlorwasserstoffs.

DE 2.847.243 schlägt die Phosgen-Entfernung durch Strippen mit gasförmigem Chlorwasserstoff oder Stickstoff bei 170°C innerhalb von 2 Stunden vor. Nachteilig sind die erheblichen Mengen an mit Phosgen bzw. mit Phosgen/Chlorwasserstoff beladenen Gase, die einen zusätzlichen Aufwand für die anschließende Stofftrennung bzw. zusätzlichen Aufwand für die Neutralisation der sauren Gasbestandteile zwingend bedingen. Der dem Verfahren gemäß DE 2.847.243 zusätzlich anhaftende Nachteil der langen Verweilzeit für das Strippen wird in der JP 07.233.136 A durch das zweistufige Strippen mit Chlorwasserstoff nach der Phosgenentfernung bei 115°C/30 Minuten und 160°C/3 Minuten teilweise behoben. Dadurch entsteht aber der Nachteil einer zusätzlichen technologischen Operation und eines wiederum deutlichem Gasanfallstroms, der einer Behandlung bedarf.

Nach JP 07.082.230 A werden dem Anilin vor der Anilin-Formaldehyd-Kondensation organische Phosphite zugesetzt.

Zur Senkung der Jodfarbzahl wird die Zugabe zahlreicher Verbindungen nach der Phosgenierung vorgeschlagen: Wasser (US 4.465.639), Phenolderivate (DE 4.300.774), Amine u./o. Harnstoffe (DE 4.232.769), Säurechloride/Chlorformiate (DE 4.118.914), Wasser (US 4.465.639), Polyoxyalkylen-polyalkohole (DE 4.021.712), Di- oder Tris-alkyl-phosphite (DE 4.006.978), niedermolekulare ein- oder mehrbasige Alkohole (EP 445.602), Säurechloride/Antioxidantiens (DE 4.318.018).

Allen Verfahren, die die Zugabe von Verbindungen zu Rohstoffen oder Produkten einer Herstellungsstufe des PMDI vorschlagen, ist der Nachteil der Zugabe eines zusätzlichen Agens mit der damit innewohnenden Gefahr dessen korrosiver Wirkung auf die Ausrüstungsteile und der Bildung von Nebenprodukten aus eben diesen zugesetzten Agenzien, die sich ihrerseits nachteilig auf das Produkt oder die Ausrüstungen auswirken können.

In US 4.876.380 wird die Farbaufhellung durch Extraktion einer chromophorenreichen PMDI-Fraktion aus dem PMDI durch Pentan/Hexan vorgeschlagen. Nachteilig an diesem Verfahren sind das Durchführen einer aufwendigen technologischen Operation mit zusätzlichen Aufarbeitungsschritten des Extraktionsmittels und dem Zwangsanfall einer qualitätsgeminderten PMDI-Fraktion, für die mengenäquivalente Anwendungen gefunden werden müssen.

Die Aufgabe der vorliegenden Erfindung bestand darin, unter Vermeidung der genannten Nachteile den Gehalt an chlorierten Nebenprodukten und die Jodfarbzahl des PMDI in Mischung mit MMDI zu vermindern, wobei insbesondere auf den Zusatz von Hilfsstoffen und/oder zusätzlicher Apparate verzichtet werden sollte.

Diese Aufgabe konnte überraschenderweise gelöst werden durch zweistufig Umsetzung der entsprechenden Mischungen enthaltend Diphenylmethan-diaminen und Polyphenylen-polymethylen-polyaminen mit Phosgen in Gegenwart mindestens eines inerten organischen Lösungsmittels, wobei die in der ersten Stufe der Phosgenierung gebildeten entsprechenden Carbamylchloride und Aminhydrochloride in der zweiten Stufe der Phosgenierung eine Kolonne als Verweilzeitapparat durchlaufen, in dem die Aminhydrochloride zu den entsprechenden Carbamylchloriden phosgeniert und die Carbamylchloride zu den entsprechenden Isocyanaten und Chlorwasserstoff gespalten werden und die Massenverhältnisse von Phosgen zu Chlorwasserstoff gleichzeitig in der Flüssigphase 10-30:1 und in der Gasphase 1-10:1 betragen.

Gegenstand der Erfindung ist somit ein Verfahren zur Herstellung von Mischungen enthaltend Diphenylmehandiisocyanaten und Polyphenylen-polymethylen-polyisocyanaten mit vermindertem Gehalt an chlorierten Nebenprodukten und verminderter Jodfarbzahl durch zweistufige Umsetzung der entsprechenden Mischungen enthaltend Diphenylmethan-diaminen und Polyphenylen-polymethylen-polyaminen mit Phosgen in Gegenwart mindestens eines inerten organischen Lösungsmittels, das dadurch gekennzeichnet ist, daß die in der ersten Stufe der Phosgenierung gebildeten Carbamylchloride und Aminhydrochloride in der zweiten Stufe der Phosgenierung eine Kolonne als Verweilzeitapparat durchlaufen, in dem die Aminhydrochloride zu den entsprechenden Carbamylchloriden phosgeniert und die Carbamylchloride zu den entsprechenden Isocyanaten und Chlorwasserstoff gespalten werden und die Massenverhältnisse von Phosgen zu Chlorwasserstoff gleichzeitig in der Flüssigphase 10-30:1 und in der Gasphase 1-10:1 betragen.

Die Phosgenierung primärer Amine in einem Mischreaktor als erste Stufe der Phosgenierung ist mehrfach beschrieben worden. So teilen z.B. US 3.544.611, EP A2-0150435 die Phosgenierung in einem Druckmischkreis mit. Auch ist die Durchführung dieser Reaktion gemäß EP A2-0291819 in einer Reaktionspumpe bekannt. Vielfältig werden unterschiedliche Ausführungen statischer Mischer beschreiben. Nur beispielhaft seien genannt: Ringschlitzdüse (FR 2.325.637, DE 1.792.660), Ringlochdüse (DE 3.744.001), Glattstrahldüse (EP A1- 0.065.727), Fächerstrahldüse (DE 2.950.216), Winkelstrahlkammerdüse (DD 300.168), Dreistromdüse (DD 132.340).

Das Durchlaufen eines Verweilzeitapparates für die in der ersten Stufe der Phosgenierung gebildeten Carbamylchloride und Aminhydrochloride, in dem die Aminhydrochloride zu den entsprechenden Carbamylchloriden phosgeniert und die Carbamylchloride zu den entsprechenden Isocyanaten und Chlorwasserstoff gespalten werden, an sich ist bekannt. Das nach WO 96/16.028 in einem Rohrreaktor bei 80-150°C hergestellte Isocyanat zeichnet sich durch einen sehr ungenügenden Wert für das hydrolysierbare Chlor von max. 2 % aus und macht nach diesem Verfahren hergestelltes PMDI für die meisten Anwendungen unbrauchbar. In BE 790.461 und BE 855.235 werden Rührapparate als Verweilzeitreaktoren benutzt. US 3.544.611 beschreibt einen bei 10-50 bar und 120-150°C arbeitenden Destillationsverweilzeitapparat mit einer "ausgedehnten Destillationssektion" ("elongated distillation zone") zur Spaltung der Carbamylchloride und Entfernung des Chlorwasserstoffs. In DE 3.744.001 wird eine von unten nach oben durchströmte Lochbodenkolonne mit größer 10 Lochböden, einer Verweilzeit von max. 120 Minuten und Flüssiggeschwindigkeiten von 0,05-4 m/s und Gasgeschwindigkeiten von 2-20 m/s vorgeschlagen. Nachteilig bei den beschriebenen Verfahren des Standes der Technik sind die drastischen Bedingungen in den Verweilzeitapparaten und der relativ langen Verweilzeit des gebildeten Roh-PMDI. Erfahrungsgemäß lassen diese Verfahren nur ein sehr unzureichendes Qualitätsniveau bezüglich der Farbe und des Chlorgehaltes im PMDI zu.

Auch die Kombination von Misch- und Verweilzeitapparat zur Herstellung von PMDI, insbesondere für die Zweistufenphosgenierung, ist bekannt. So wird in DE 3.744.001 eine Ringlochdüse als Reaktor der Umsetzung von primären Aminen mit Phosgen in einem inerten Lösungsmittel zu den entsprechenden Carbamylchloriden und Aminhydrochloriden mit einer oder mehreren Lochbodenkolonnen als Apparat zur Phosgenierung der Aminhydrochloride und Carbamylchloridspaltung kombiniert. US 3.381.025 führt die erste Stufe bei < 60°C in einem inerten Lösungsmittel mit einem Siedepunkt von 100-190°C aus und überführt das Reaktionsprodukt in eine zweite Stufe, in der die Temperatur so über dem Siedepunkt des inerten Lösungsmittel gehalten wird, daß das Mengenverhältnis des entweichenden Phosgens zum inerten Lösungsmittel größer zwei ist und ggf. zusätzlich Phosgen in die zweite Reaktionsstufe eingespeist wird. Nachteilig sind der hohe apparative bzw. energetische Aufwand für die zweite Stufe der Phosgenierung als Verweilzeitapparat bzw. zur Kondensation des gasförmigen Gemisches Phosgen/inertes Lösungsmittel. Ein derartiges Verfahren läßt nur ein sehr unzureichendes Qualitätsniveau bezüglich des Chlorgehaltes und der Farbe im PMDI zu.

Deshalb bestand eine weitere Aufgabe der vorliegenden Erfindung darin, den Gehalt an chlorierten Nebenprodukten und die Jodfarbzahl des PMDI unter Verwendung sicherheitstechnisch und apparativ einfacher technologischer Ausrüstungen zu vermindern.

Diese Aufgabe konnte überraschenderweise gelöst werden durch zweistufige Umsetzung des PMDA mit Phosgen in Gegenwart mindestens eines inerten organischen Lösungsmittels, wobei die erste Stufe der Phosgenierung in einem statischen Mischer und die zweite Stufe der Phosgenierung in eine Kolonne als Verweilzeitapparat durchgeführt werden und in der Kolonne die Massenverhältnisse von Phosgen zu Chlorwasserstoff gleichzeitig in der Flüssigphase 10-30:1 und in der Gasphase 1-10:1 betragen.

Als statische Mischer für die erste Stufe der Phosgenierung kommen die bekannten und oben aufgeführten Vorrichtungen, insbesondere Düsen, zur Anwendung. Die Temperatur bei der ersten Stufe des Phosgenierung beträgt üblicherweise 40 bis 150°C, bevorzugt 60 bis 130°C, besonders bevorzugt 90-120°C.

Das Gemisch der ersten Stufe der Phosgenierung wird einer Kolonne zugeführt, wobei erfindungsgemäß die Massenverhältnisse von Phosgen zu Chlorwasserstoff in der Kolonne der zweiten Stufe der Phosgenierung gleichzeitig in der Flüssigphase 10-30:1 und in der Gasphase 1-10:1 betragen.

Bei dem erfindungsgemäßen Verfahren ist es besonders vorteilhaft, die Kolonne im Gegenstrom zu betreiben. Das Produktgemisch der ersten Stufe der Phosgenierung wird bevorzugt so in die Kolonne eingespeist,daß das PMDI/Lösungsmittel/Phosgen-Gemisch die Kolonne über den Sumpf verläßt und ein Phosgen/Chlorwasserstoffgemisch über Kopf der Kolonne abgezogen wird und der Chlorwasserstoff/Phosgen-Trennung zugeführt wird. Die Eintrittstemperatur des Gemisches der ersten Stufe der Phosgenierung in die Kolonne kann bevorzugt 80-120°C, besonders bevorzugt 82-117°C, betragen. Die Sumpftemperatur der Kolonne beträgt dabei vorzugsweise 80-120°C, besonders bevorzugt 90-110°C. Der Kopfdruck der Kolonne beträgt vorzugsweise 1,0-4,7 at (Ü), besonders bevorzugt 2,0-3,7 at (Ü). Das Chlorwasserstoff/Phosgenverhältnis in der Kolonne wird durch den Phosgenüberschuß in der ersten Stufe der Phosgenierung, die Eintrittstemperatur des Reaktionsproduktes in die Kolonne, den Kolonnendruck und die Sumpftemperatur der Kolonne eingestellt und kontrolliert. Die Phosgenmenge kann insgesamt der ersten Stufe der Phosgenierung zugeführt werden oder nur teilweise, wobei in diesem Fall eine weitere Phosgenmenge in den Verweilzeitapparat der zweiten Stufe der Phosgenierung eingespeist wird. Die verwendete Kolonne hat vorzugsweise < 10 theoretischen Böden. Vorteilhaft ist die vorzugsweise Verwendung einer Ventilbodenkolonne. Es sind auch andere Kolonneneinbauten geeignet, die die notwendige Verweilzeit für die Carbamylchloridspaltung sowie eine schnelle und effektive Chlorwasserstoffentfernung gewährleisten, wie z.B. Glockenbodenkolonnen, Destillationsböden mit erhöhten Flüssigkeitswehren. Die in DE-A 3 744 001 vorgeschlagene Lochbodenkolone kann die Aufgabe der schonenden Carbamylchloridspaltung bei schneller und effektiver Chlorwasserstoffentfernung technisch nur sehr unzureichend erfüllen und ist als Verweilzeitapparat wegen ihres Gleichstromprinzipes, was zwangsläufig zu einem großen Flüssigkeitsholdups und zur erschwerten schnellen Chlorwasserstoffentfernung führt, zur Herstellung eines PMDI mit vermindertem Chlorgehalt und verminderter Jodfarbzahl ungeeignet.

Die nach dem erfindungsgemäßen Verfahren hergestellten Mischungen aus Diphenylmehandiisocyanaten und Polyphenylen-polymethylenpolyisocyanaten besitzen üblicherweise einen Diphenylmethandiisocyanat-Isomerengehalt von 30 bis 90 Ma.-%, vorzugsweise von 30 bis 70 Gew.-%, einen NCO-Gehalt von 29 bis 33 Gew.-%, vorzugsweise 30 bis 32 Ma.-%, bezogen auf das Roh-MDI-Gewicht, und eine Viskosität, bestimmt gemäß DIN 51550 bei 25°C, von maximal 2500 mPa.s, vorzugsweise von 40 bis 2000 mPa.s.

Für das erfindungsgemäße Verfahren geeignete Roh-MDA werden vorteilhafterweise erhalten durch Kondensation von Anilin und Formaldehyd in einem Molverhältnis von 6 bis 1,6:1, vorzugsweise von 4 bis 1,9:1, und einem Molverhältnis von Anilin zu sauren Katalysatoren von 1:0.98 bis 0,01, vorzugsweise 1:0,8 bis 0,1.

Der Formaldehyd wird vorzugsweise in Form einer wäßrigen Lösung, z.B. als handelsübliche 30 bis 50 ma.-%ige Lösung, verwendet.

Als saure Katalysatoren haben sich Protonendonatoren, wie z.B. saure Ionenaustauscherharze oder starke organische und vorzugsweise anorganische Säuren bewährt. Als starke Säuren sind hierbei solche mit einem pKs-Wert kleiner als 1,5, bei mehrbasischen Säuren gilt dieser Wert für die erste Wasserstoffdissoziation, zu verstehen. Beispielhaft genannt seien Salzsäure, Schwefelsäure, Phosphorsäure, Fluorsulfonsäure und Oxalsäure. Chlorwasserstoff kann auch gasförmig eingesetzt werden. Vorzugsweise zur Anwendung kommt wäßrige Salzsäure in Konzentrationen von etwa 25 bis 33 Ma.-%.

In Betracht kommende Verfahren zu Roh-MDA-Herstellung werden beispielsweise beschrieben in CA-A-700 026, DE-B-22 27 110 (US-A-4 025 557), DE-B-22 38 920 (US-A-3,996,283), DE-B-24 26 116 (GB-A-1,450,632), DE-A-12,42,623 (US-A-3,478,099), GB-A-1,064,559 und DE-A-32 25 125.

Als andere Ausgangskomponente zur Herstellung von Roh-MDI wird Phosgen verwendet. Das gasförmige Phosgen kann als solches oder in Verdünnung mit unter den Reaktionsbedingungen inerten Gasen, wie Stickstoff, Kohlenmonoxid u.a. eingesetzt werden. Das Molverhältnis von Roh-MDA zu Phosgen wird zweckmäßigerweise so bemessen, daß pro Mol NH2-Gruppe 1 bis 10 Mol, vorzugsweise 1,3 bis 4 Mol, Phosgen in der Reaktionsmischung vorliegen. Die Phosgenmenge kann vollständig der ersten Stufe der Phosgenierung zugeführt werden oder teilweise auch dem Verweilzeitapparat der zweiten Stufe der Phosgenierung zugesetzt werden.

Als inerte organische Lösungsmittel kommen Verbindungen in Betracht, in welchen das Roh-MDA und das Phosgen mindestens teilweise löslich sind.

Als Lösungsmittel vorzüglich bewährt haben sich chlorierte, aromatische Kohlenwasserstoffe, beispielsweise Monochlorbenzol, Dichlorbenzole wie z.B. o-Dichlorbenzol, p-Dichlorbenzol, Trichlorbenzole, die entsprechende Toluole und Xylole, Chlorethylbenzol, Monochlordiphenyl, alpha- bzw. beta-Naphthylchlorid und Phthalsäuredialkylester, wie iso-Diethylphthalat. Insbesondere Anwendung finden als inerte organische Lösungsmittel Monochlorbenzol, Dichlorbenzole oder Mischungen dieser Chlorbenzole. Die Lösungsmittel können einzeln oder als Gemische verwendet werden. Zweckmäßigerweise wird ein Lösungsmittel verwendet, das einen niedrigeren Siedepunkt besitzt als die MDI-Isomeren, damit das Lösungsmittel leicht durch Destillation vom Roh-MDI abgetrennt werden kann. Die Menge an Lösungsmittel wird zweckmäßig so bemessen, daß die Reaktionsmischung einen Isocyanatgehalt von 2 bis 40 Ma.-%, vorzugsweise zwischen 5 und 20 Ma.-%, bezogen auf das Gesamtgewicht der Reaktionsmischung, aufweist.
Das Roh-MDA kann als solches oder gelöst in organischen Lösungsmitteln zur Anwendung kommen. Insbesondere verwendet man jedoch Roh-MDA-Lösungen mit einem Amingehalt von 2 bis 45 Ma.-%, vorzugsweise von 25 bis 44 Ma.-%, bezogen auf das Gesamtgewicht der Aminlösung.

Im Anschluß an die Phosgenierung werden bevorzugt das überschüssige Phosgen, der Chlorwasserstoff und das Lösungsmittels vom Reaktionsprodukt abgetrennt. Für die Herstellung eines PMDI mit verringertem Gehalt an chlorierten Nebenprodukten und verringerter Jodfarbzahl ist es besonders vorteilhaft, daß der Restgehalt an Phosgen nach der Phosgenentfernung < 10 ppm Phosgen beträgt. Diese Aufarbeitungsschritte erfolgen nach den allgemein bekannten Verfahren. Aus dem MDI-Gemisch können durch bekannte Verfahren wie Destillation oder Kristallisation die Zweikern-Isomere abgetrennt werden.

Danach wird es üblicherweise mit einem Antioxidans auf Basis sterisch gehinderter Phenole und/oder mindestens einem Arylphosphit stabilisiert. Die Stabilisatoren werden zweckmäßigerweise in einer Menge bis max. 1 Ma.-%, vorzugsweise von 0,001 bis 0,2 Ma.-% eingesetzt.

Als geeignete Antioxidantien auf Basis sterisch gehinderter Phenole kommen beispielsweise in Betracht: Styrolisierte Phenole, d.h. Phenole, die in 2- oder 4-Stellung oder in 2- und 4- und/oder 6-Stellung eine 1-Phenyl-ethylgruppe gebunden enthalten, Bis-[2-hydroxy-5-methyl-3-tertbutylphenyl-]-methan, 2,2-Bis-[4-hydroxyphenyl]-propan, 4,4'-Dihydroxy-biphenyl, 3,3'-Dialkyl- bzw. 3,3', 5,5'-Tetraalkyl-4,4'-dihydroxy-biphenyl, Bis-[4-hydroxy-2-methyl-5-tert.-butylphenyl]-sulfid, Hydroxinon, 4-Methoxy-, 4-tert.-Butoxy- oder 4-Benzyloxyphenol, Gemische aus 4-Methoxy-2- bzw. -3-tert.-butylphenol, 2,5-Dihydroxy-1-tert.butylbenzol, 2,5-Dihydroxy-1,4-di-tert.-butylbenzol, 4-Methoxy-2,6-di-tert.-butylphenol und vorzugsweise 2,6-Di-tertbutyl-p-kresol.

Als Arylphosphite bewährt haben sich Tri-(alkylphenyl)-phosphite mit 1 bis 10 C-Atomen im Alkylrest, wie z.B. Tri-(methylphenyl)-, Tri-(ethylphenyl)- Tri-(n-propylphenyl)-, Tri-(isopropylphenyl)-, Tri-(n-butylphenyl)-, Tri-(sek.-butylphenyl), Tri-(tert.-butylphenyl, Tri-(pentylphenyl)-, Tri-(hexylphenyl)-, Tri-(2-ethyl-hexylphenyl)-, Tri-(oktylphenyl)-,Tri-(2-ethyl-octylphenyl)-, Tri-(decylphenyl)-phosphit und vorzugsweise Tri-(nonylphenyl)-phosphit, und insbesondere Triphenylphosphit.

Danach werden die auf diese Weise hergestellten Roh-PMDI üblicherweise einer thermischen Nachbehandlung, die mit der Abtrennung der MMDI-Isomeren gekoppelt sein kann, unterworfen. Dazu wird das PMDI auf eine Temperatur von 170-230 °C, vorzugsweise von 180-220°C, erhitzt und bei dieser Temperatur unter einem Druck von 0,01 bis 100 mbar, vorzugsweise von 0,1 bis 20 mbar, mindestens 5 Minuten und insbesondere 5 bis 45 Minuten, gegebenenfalls unter Einleitung einer Menge von max. 5 Nm³/t PMDI eines Inertgases, z.B. Stickstoff, vorzugsweise max. 0,5 Nm³/t PMDI Inertgas, behandelt.

Nach der Abkühlung auf 30-60°C wird das PMDI üblicherweise der Zwischenlagerung zugeführt.

Die Erfindung soll an nachfolgenden Beispielen näher erläutert werden:

### Beispiel 1:

Zur Phosgenierung wird ein PMDA folgender Zusammensetzung eingesetzt:
- Viskosität bei 70°C 348 mm²/s
- Gehalt an 4,4'-Diphenylmethandiamin (4,4'-MDA) 44,6 Masse-%
- Gehalt an MDA 52 Masse-%
- Gehalt an 3-Kern-PMDA 23 Masse-%
- Gehalt an N-Methyl-MDA 0,14 Masse-%
- Gehalt an N-Formyl-MDA 1194 ppm.

3.840 kg/h eines solchen PMDA als 38,7 Ma.-%-ige Lösung in Monochlorbenzol (MCB) werden mit 26.400 kg/h einer 42 Ma-%-igen Lösung von Phosgen in MCB in einer Winkelstrahlkammerdüse phosgeniert. Das Reaktionsgemisch erhitzt sich im Reaktor der ersten Stufe der Phosgenierung durch die Exothermie der Reaktion von PMDA mit Phosgen auf eine Temperatur von 118°C und tritt mit 92°C in eine Ventilbodenkolonne mit 6 theoretischen Böden im Abtriebsteil und 2 Böden im Verstärkerteil ein. Die Kolonne wird bei einem Druck von 4,3 bar (abs.) betrieben und die Sumpfzusammensetzung durch Wahl der Heizdampfmenge so eingestellt, daß der Phosgengehalt im Sumpf der Kolonne ca. 10 Ma.-% beträgt, was einer Temperatur im Sumpf der Kolonne von 95-97°C entspricht. Die Masseverhältnisse von Phosgen zu Chlorwasserstoff betragen im Sumpf der Kolonne 14,2:1 und im Kopf der Kolonne 1,6:1. Der in der ersten Stufe der Phosgenierung gebildete und in der Kolonne aus der Spaltung der Carbamylchloride freigesetzte Chlorwasserstoff wird zusammen mit einem Teil des im Überschuß eingesetzten Phosgens über Kopf bei einer Temperatur von 91°C abgezogen. Um das Mitreißen von PMDI-Tröpfchen zusammen mit dem Chlorwasserstoffund Phosgengasströmen zu verhindern, wird in den Kopf der Kolonne zusätzlich MCB in einer Menge von 1.350 kg/h eingespeist.

Das die Phosgenierung verlassende Gemisch wird entsprechend dem Stand der Technik vom Phosgen und MCB befreit und thermisch nachbehandelt.

Das so hergestellte PMDI wird durch folgende Produkteigenschaften charakterisiert:
- Viskosität bei 25°C nach DIN 51550 182 mPa*s
- Gehalt an Isocyanatgruppen nach ASTM D 1638-74 31,5 Ma-%
- Acidität nach ASTM D 1638-74 56 ppm HCl
- Totalchlor nach DIN 35474 900 ppm HCl
- Jodfarbzahl ¹⁾ 9,7

¹⁾ Gemessen mit Dreifiltergerät, z.B. LICO 200 (Fa. Dr. Lange)

### Vergleichsbeispiel 1:

Zum Vergleich wird das gleiche PMDA wie in Beispiel 1 in der gleichen Winkelstrahlkammerdüse und gleicher Kolonne phosgeniert. Ebenfalls werden 3.840 kg/h diesen PMDA als 38,7 Ma.-%-ige Lösung in Monochlorbenzol (MCB) mit 26.400 kg/h einer 42 Ma-%-igen Lösung von Phosgen in MCB zur Reaktion gebracht. Ebenfalls wird in den Kopf der Kolonne zusätzlich MCB in einer Menge von 1.350 kg/h eingespeist.

Die Eintrittstemperatur des PMDA/MCB-Stroms zur Winkelstrahlkammerdüse wird so gewählt, daß die Temperatur des die Düse verlassenden Reaktionsgemisches 96°C beträgt. Das Reaktionsgemisch tritt mit 78°C in die Ventilbodenkolonne ein. Die Kolonne wird bei einem Kopfdruck von 5,2 bar (abs.) betrieben. Bei einer auf 116°C eingestellten Sumpftemperatur stellt sich eine Kopftemperatur von 76°C ein. Die Masseverhältnisse von Phosgen zu Chlorwasserstoff betragen im Sumpf der Kolonne 9,2:1 und im Kopf der Kolonne 0,95:1.

Das als Vergleich hergestellte PMDI besitzt folgende Produkteigenschaften:
- Viskosität bei 25°C nach DIN 51550 197 mPa*s
- Gehalt an Isocyanatgruppen nach ASTM D 1638-74 31,8 Masse-%
- Acidität nach ASTM D 1638-74 197 ppm HCl
- Totalchlor nach DIN 35474 1900 ppm HCl
- Jodfarbzahl ¹⁾ 15

¹⁾ Gemessen mit Dreifiltergerät, z.B. LICO 200 (Fa. Dr. Lange)

## Patentansprüche

1. Verfahren zur Herstellung von Mischungen enthaltend Diphenylmehandiisocyanate und Polyphenylen-polymethylen-polyisocyanate, mit vermindertem Gehalt an chlorierten Nebenprodukten und verminderter Jodfarbzahl durch zweistufige Umsetzung der entsprechenden Mischungen enthaltend Diphenylmethan-diamine und Polyphenylen-polymethylen-polyamine mit Phosgen in Gegenwart mindestens eines inerten organischen Lösungsmittels, **dadurch gekennzeichnet, daß** die Massenverhältnisse von Phosgen zu Chlorwasserstoff im Verweilzeitapparat der zweiten Stufe der Phosgenierung gleichzeitig in der Flüssigphase 10-30:1 und in der Gasphase 1-10:1 betragen.

2. Verfahren nach Anspruch 1, **dadurch gekennzeichnet, daß** als Apparat der ersten Stufe der Phosgenierung ein statischer Mischer mit einer Gemischaustrittstemperatur von 80-120°C verwendet wird.

3. Verfahren nach Anspruch 1, **dadurch gekennzeichnet, daß** als Apparat der zweiten Stufe der Phosgenierung eine Kolonne mit < 10 theoretischen Böden benutzt wird.

4. Verfahren nach Anspruch 3, **dadurch gekennzeichnet, daß** die Kolonne im Gegenstrom betrieben wird.

5. Verfahren nach Anspruch 3, **dadurch gekennzeichnet, daß** es sich um eine Ventilbodenkolonne handelt.

6. Verfahren nach Anspruch 3, **dadurch gekennzeichnet, daß** es sich um eine Glockenbodenkolonne handelt.

7. Verfahren nach Anspruch 3, **dadurch gekennzeichnet, daß** die Kolonne Destillationsböden mit erhöhten Flüssigkeitswehren aufweist.

8. Verfahren nach Anspruch 1, **dadurch gekennzeichnet, daß** die PMDA-Konzentration im inerten Lösungsmittel im Strom zum statischen Mischer max. 44 Ma.-% beträgt.

9. Verfahren nach Anspruch 3, **dadurch gekennzeichnet, daß** die Eintrittstemperatur des Gemisches der ersten Stufe der Phosgenierung in die Kolonne 80-120°C, bevorzugt 82-117°C, beträgt.

10. Verfahren nach Anspruch 3, **dadurch gekennzeichnet, daß** die Sumpftemperatur der Kolonne 80-120°C, bevorzugt 90-110°C, beträgt.

11. Verfahren nach Anspruch 3, **dadurch gekennzeichnet, daß** der Kopfdruck der Kolonne 1,0-4,7 at (Ü), bevorzugt 2,0-3,7 at (Ü), beträgt.

## Claims

1. A process for preparing mixtures comprising diphenylmethane diisocyanates and polyphenylpolymethylene polyisocyanates having a reduced content of chlorinated by-products and a reduced iodine color number by two-stage reaction of the corresponding mixtures comprising diphenylmethanediamines and polyphenylpolymethylenepolyamines with phosgene in the presence of at least one inert organic solvent, wherein the mass ratios of phosgene to hydrogen chloride in the residence time apparatus of the second stage of the phosgenation are at the same time 10-30:1 in the liquid phase and 1-10:1 in the gas phase.

2. A process as claimed in claim 1, wherein the apparatus used in the first stage of the phosgenation is a static mixer having a mix exit temperature of 80-120°C.

3. A process as claimed in claim 1, wherein the apparatus used in the second stage of the phosgenation is a column having < 10 theoretical plates.

4. A process as claimed in claim 3, wherein the column is operated in countercurrent.

5. A process as claimed in claim 3, wherein the column is a valve tray column.

6. A process as claimed in claim 3, wherein the column is a bubble cap tray column.

7. A process as claimed in claim 3, wherein the column has distillation trays having relatively high liquid weirs.

8. A process as claimed in claim 1, wherein the PMDA concentration in the inert solvent in the stream to the static mixer is at most 44% by mass.

9. A process as claimed in claim 3, wherein the temperature at which the mixture from the first stage of the phosgenation enters the column is 80-120°C, preferably 82-117°C.

10. A process as claimed in claim 3, wherein the temperature at the bottom of the column is 80-120°C, preferably 90-110°C.

11. A process as claimed in claim 3, wherein the pressure at the top of the column is 1.0-4.7 atm (gauge pressure), preferably 2.0-3.7 atm (gauge pressure).

## Revendications

1. Procédé de préparation de mélanges contenant des diphénylméthane-diisocyanates et des polyphénylène-polyméthylène-polyisocyanates, ayant une teneur réduite en sous-produits chlorés et un indice de teinte d'iode réduit, par une réaction en deux étapes des mélanges correspondants contenant des diphénylméthane-diamines et des polyphénylène-polyméthylène-polyamines avec du phosgène en présence d'au moins un solvant organique inerte, **caractérisé en ce que** les rapports massiques du phosgène à l'acide chlorhydrique dans l'appareil où se déroule la seconde étape de la phosgénation sont simultanément, dans la phase liquide, de 10-30 :1 et, dans la phase gazeuse, de 1-10 :1.

2. Procédé selon la revendication 1, **caractérisé en ce qu'**on utilise comme appareil de la première étape de phosgénation un mélangeur statique ayant une température de sortie du mélange de 80 à 120°C.

3. Procédé selon la revendication 1, **caractérisé en ce qu'**on utilise comme appareil de la seconde étape de la phosgénation une colonne ayant moins de 10 plateaux théoriques.

4. Procédé selon la revendication 3, **caractérisé en ce qu'**on fait fonctionner la colonne à contre-courant.

5. Procédé selon la revendication 3, **caractérisé en ce qu'**il s'agit d'une colonne avec plateau à clapet.

6. Procédé selon la revendication 3, **caractérisé en ce qu'**il s'agit d'une colonne à plateau en cloche.

7. Procédé selon la revendication 3, **caractérisé en ce que** la colonne présente des plateaux de distillation à déversoirs de liquide surélevés.

8. Procédé selon la revendication 1, **caractérisé en ce que** la concentration de PMDA dans le solvant inerte dans le courant menant au mélangeur statique s'élève au maximum à 44 % en masse.

9. Procédé selon la revendication 3, **caractérisé en ce que** la température d'entrée du mélange de la première étape de la phosgénation dans la colonne s'élève à 80-120°C, de préférence à 82-117°C.

10. Procédé selon la revendication 3, **caractérisé en ce que** la température du fond de la colonne s'élève à 80-120°C, de préférence à 90-110°C.

11. Procédé selon la revendication 3, **caractérisé en ce que** la température de tête de la colonne s'élève à 1,0-4,7 atm (rel.), de préférence à 2,0-3,7 atm (rel.).
